# EUROPEAN PATENT SPECIFICATION

(11) **EP 2 382 296 B1**
(45) Date of publication and mention of the grant of the patent: **04.11.2015**
(21) Application number: 09839014.9
(22) Date of filing: 21.07.2009
(51) Int. Cl.: C11D 1/12, C11D 3/00

(54) **LIGHT DUTY LIQUID DETERGENT COMPOSITIONS OF SULFONATED ESTOLIDES AND OTHER DERIVATIVES OF FATTY ACIDS AND USES THEREOF**
FLÜSSIGE FEINWASCHMITTEL MIT SULFONATGRUPPENHALTIGEN ESTOLIDEN UND ANDEREN FETTSÄUREDERIVATEN SOWIE IHRE VERWENDUNG
COMPOSITIONS DÉTERGENTES LIQUIDES D'ESTOLIDES SULFONÉS ET AUTRES DÉRIVÉS D'ACIDES GRAS POUR LAVAGES DÉLICATS ET LEURS UTILISATIONS

(30) Priority: 21.01.2009 WO PCT/US2009/031608
(43) Date of publication of application: 02.11.2011
(73) Proprietor: STEPAN COMPANY, Northfield, Illinois 60093 (US)
(72) Inventor: BERNHARDT, Randal, J., Antioch IL 60002 (US); ALONSO, Lourdes, R., Deerfield 60015 (US); DADO, Gregory, P., Chicago, IL 60657 (US)
(74) Representative: Schnappauf, Georg
(86) International application number: PCT/US2009/051319
(87) International publication number: WO 2010/085279

(56) References cited:
- WO-A1-00/18363
- WO-A1-01/53247
- GB-A- 1 047 772
- GB-A- 1 380 390
- A.J. STIRTON, ET AL.: "Surface-active properties of salts of alpha-sulphonated acids and esters" JOURNAL OF THE AMERICAN OIL CHEMISTS' SOCIETY, vol. 13, no. 1, 1 January 1954 (1954-01-01), pages 13-16, XP002537683 SPRINGER, BERLIN ISSN: 0003-021X

## Description

### FIELD OF THE INVENTION

The present technology, in general, relates to sulfo-estolides. More particularly, the present technology relates to light duty liquid (LDL) detergent compositions that contain sulfo-estolides as surfactants. The sulfo-estolide surfactants include sulfo-estolide derivatives and salts of sulfo-estolides. Applications and/or processes of utilizing the presently described sulfo-estolide surfactants, in particular as a component within light duty liquid detergent compositions and/or formulations, are also disclosed.

### BACKGROUND OF THE INVENTION

Stirton et al. (The Journal of the American Oil Chemists' Society 31 (1954), 13-16) describe the surface-active properties of salts of alpha-sulfonated acids and esters. GB 1 380 390 discloses detergent solid and liquid formulations which are intended for the purposes of fabric washing. WO 00/18363 relates to shampoos and conditioners containing estolides.

Desirable attributes for light duty liquid detergents, in general, include the ability to emulsify, suspend or penetrate greasy or oily soils and suspend or disperse particulates, in order to clean articles or surfaces; and then prevent the soils, grease, or particulates from re-depositing on the newly cleaned articles or surfaces. It is also desirable for the light duty liquid to provide sustained foaming in dilute wash solution in the presence of the soils being cleaned. In order to optimize these attributes, it is desirable to produce LDLs that contain moderate to high levels of surfactants (e.g., greater than about 20% total surfactant) In combinations and types that would typically produce gels instead of liquids. For example, such gels were not workable, not easily dispensed or poured, at room temperature. Surprisingly, the present technology now demonstrates that the addition of at least one sulfo-estolide surfactant, having the general Formula 1 as described herein, to LDL formulations, for example, decreases the viscosity of such a formulation into a workable liquid range at room temperature. Further, the sulfo-estolide containing LDL formulations of the present technology maintain high foaming and optimized cleaning attributes.

In addition, there is the further challenge of developing environmentally friendly or "green" light duty liquid detergents as state and federal regulations are restricting the amount and use of phosphates in such detergents. The desirability of avoiding phosphates in detergents is well recognized, and phosphorus compounds have been banned from laundry detergents for many years though other detergents have been exempted from the phosphate ban on the basis that such phosphates are necessary for acceptable washing performance. Phosphorus-based compounds when released into water sources such as lakes, rivers, and bays, serve as nutrients for algae growth, resulting in deterioration of water quality. The algae blooms in lakes and ponds can suffocate plants and animals that live in those bodies of water and seriously disrupt the quality of waterways. Therefore, there has been the continuing challenge to develop and formulate "green" formulations of light duty liquid detergents that provide adequate foaming and cleaning capabilities, but with reduced or prevented negative environmental impact. The sulfo-estolide surfactants/compositions/components disclosed herein and/or light duty liquid detergent formulations containing such sulfo-estolide components/surfactants surprisingly and unpredictably provide "green" and/or "eco-friendly" compositions that are plant derived, biodegradable and while achieving adequate foaming and cleansing.

### BRIEF SUMMARY OF THE INVENTION

In at least one aspect the present technology provides a liquid detergent composition, comprising 0.1% to 99.9% active weight of the total composition of at least one sulfo-estolide surfactant having the following general Formula 1: 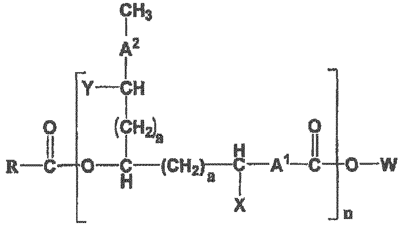 wherein n is an integer from 1-30; one of X and Y is SO₃-Z, the other of X and Y is H, and X and Y are independently assigned in each repeating unit; A¹ and A² are linear or branched, saturated or unsaturated, substituted or unsubstituted, alkyl diradicals wherein the total number of carbons for each repeating unit is independent and in the range of C₈ to C₂₂; a is 0, 1, or 2, and is independently assigned in each repeating unit; R is linear or branched, saturated or unsaturated, substituted or unsubstituted hydrocarbon wherein the total number of carbon atoms is from 1 to 24; W is H or a monovalent or divalent metal cation, ammonium cation, substituted ammonium cation, or an alkyl or substituted alkyl group; and Z is H or a monovalent or divalent metal cation, ammonium or substituted ammonium cation; and 0.1 % to 99.9% of at least one additional surfactant.

In one embodiment, the composition further comprises 0% to 40% active weight of the total composition of at least one foam stabilizing surfactant and/or 1% to 99% of at least one carrier. The at least one foam stabilizing surfactant may be 0.5% to 15% by active weight of the composition, such as 3% to 10% by active weight of the composition or 5% by active weight of the composition. In one embodiment, the at least one foam stabilizing surfactant is a member selected from the group consisting of at least one ampholytic or amphoteric surfactant (such as a member selected from the group consisting of amine oxides, amidopropyl amine oxides, betaines, amidopropyl betaines, sulfobetaines, hydroxysultaines, amphoacetates, amphopropionates, alkyl amines, organic diamines, and combinations thereof), at least one nonionic surfactant (such as a member selected from the group consisting of alcohol ethoxylates, alkyl polyglucosides, alkyl ethanolamides, alkyl esters, and combinations thereof), and combinations thereof. In an alternative embodiment, the at least one foam stabilizing surfactant is a member selected from the group consisting of cocoamidopropyl betaine, lauryl myristal amidopropyl dimethyl amine oxide, lauryl dimethyl amine oxide, alkyl polyglucoside, and combinations thereof.

In any of the above embodiments, the at least one sulfo-estolide surfactant having the general Formula 1 may be 0.1% to 50% active weight of the total composition, such as 0.1% to 30% active weight of the total composition, 0.1 % to 10% active weight of the total composition, or 1% to 10% active weight of the total composition.

In any of the above embodiments, the composition may be non-toxic, biodegradable, and substantially free of phosphates.

In any of the above embodiments, the at least one additional surfactant may be 2% to 70% active weight of the total composition, such as 5% to 45% active weight of the total composition or 10% to 30% active weight of the total composition.

In any of the above embodiments, the at least one additional surfactant may be a member selected from the group consisting of at least one anionic surfactant (such as a member selected from the group consisting of sulfoacetates, olefin sulfonates, alkyl benzene sulfonates, alkyl sulfosuccinates, alkyl sulfomethylsuccinates, and combinations thereof), at least one nonionic surfactant (such as a member selected from the group consisting of alcohol ethoxylates, alkyl polyglucosides, alkyl ethanolamides, alkyl esters, and combinations thereof), and combinations thereof. Alternatively, the at least one additional surfactant may be a member selected from the group consisting of lauryl 2-mole average ether sulfonate, lauryl 1-mole average ether sulfonate, lauryl 3-mole average ether sulfonate, alcohol ethoxylate, sodium alkylbenzenesulfonate, alkylbenzene sulfonic acid neutralized with sodium, potassium, ammonium and/or magnesium, sodium olefin sulfonate, and/or sodium lauryl sulfate.

In any of the above embodiments, the pH of the composition may be from 3 to 10, such as from 4 to 9 or from 6 to 8.

In any of the above embodiments, the composition may further comprise at least one antimicrobial ingredient, wherein the at least one antimicrobial ingredient (such as a member selected from the group consisting of triclosan, n-alkyl dimethyl benzyl ammonium chloride, dialkyl dimethyl ammonium chloride, didecyl dimethyl ammonium chloride, dioctyl dimethyl ammonium chloride, phenolics, iodophors, pine oil, methyl salicylate, morpholine, silver, copper, bromine, quaternary ammonium compounds, and combinations thereof).

In any of the above embodiments, the composition may further comprise from 0% to 20% by weight of at least one additive. In one embodiment, the at least one additive is a member selected from the group consisting of solubilizing agents, fragrances, dyes, enzymes (such as a member selected from the group consisting of proteases, amylases, lipases, and combinations thereof), preservatives (such as a member selected from the group consisting of benzyl alcohol, phenoxy-2-ethanol, methyl paraben, propyl paraben, methylchloroisothiazolinone, methylisothiazolinone, 2-methyl-4-isothiazolin-3-one, 1,2-benzisothiazolin-3-one, imidazolidinyl urea, 1,3-dimethylol-5,5-dimethylhydantoin, and combinations thereof), polymers (such as a member selected from the group consisting of anionic polymers (e.g., acrylates and combinations thereof), hydroxyethylcelluloses, zwitterionic polymers, gelatins, xanthan gums, polysaccharides, polyethylene glycols, and combinations thereof), thickeners, builders, magnesium sulfate, and combinations thereof.

In any of the above embodiments, the liquid detergent composition may further comprise at least one enzyme.

### DETAILED DESCRIPTION OF THE INVENTION

The present technology, in general, relates to sulfo-estolides. More particularly, the present technology relates to light duty liquid detergent compositions that contain sulfo-estolides as surfactants. The sulfo-estolide surfactants described herein include, but are not limited to, sulfo-estolide derivatives and salts of sulfo-estolides. Such compositions having the general Formula 1:

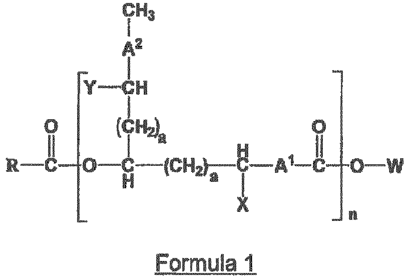

In Formula 1:
n is an integer from 1 to about 30, alternatively 1 to 10, alternatively 1 to 4, alternatively 1, 2, or 3, alternatively 1 or 2, alternatively 1; or mixtures thereof;
one of X and Y is SO₃⁻Z, the other of X and Y is H (i.e., a hydrogen atom), and X and Y are Independently assigned in each repeating unit;
A¹ and A² are independently selected linear or branched, saturated or unsaturated, substituted or unsubstituted alkyl diradicals, where the total number of carbons for each repeating unit is independent and in the range of C₈ to C₂₂. As defined here, the term "alkyl diradical" is meant to refer to a linking hydrocarbon or alkylene segment, for example but by no means limited to -(CH₂)₃-, -(CH₂)₄-, -(CH₂)₅-, and so forth;
a is 0, 1, or 2, and is independently assigned in each repeating unit. When a = 0, 1, or 2, the functional group corresponds to an alpha-sulfo-estolide, beta-sulfo-estolide, or gamma-sulfo-estolide, respectively;
R can be linear or branched, saturated or unsaturated, substituted or unsubstituted hydrocarbon, wherein the total number of carbon atoms is from 1 to 24. In at least one embodiment, R has from 7 to 21 carbon atoms, alternatively from 8 to 16 carbon atoms, and can be a saturated or unsaturated linear or branched hydrocarbon, a linear or branched hydroxyalkane sulfonate, or a linear or branched alkene sulfonate. For example, in one embodiment, A¹ and A² are linear alkyl diradicals and R is saturated or unsaturated linear hydrocarbon, linear hydroxyalkane sulfonate, or linear alkene sulfonate having from 7 carbon atoms to 21 carbon atoms, alternatively from 8 carbon atoms to 16 atoms carbons;
W is a monovalent or divalent metal; ammonium; substituted ammonium; H (i.e., a hydrogen atom); or a linear or branched, substituted or unsubstituted alkyl having from 1 to 22 carbon atoms. For example, W can be an alkali or alkaline earth metal cation. Alternatively, W can be a glycerine joined by an ester linkage, e.g., a substituted C3 alkyl such that the general Formula 1 is incorporated one or more times as an ester in a monoglyceride, a diglyceride, or a triglyceride; and
Z is H (i.e., a hydrogen atom) or a monovalent or divalent metal cation, ammonium or substituted ammonium cation, preferably an alkali or alkaline earth metal cation, for example potassium, sodium, calcium, or magnesium, with potassium being preferred in certain embodiments.

The above structure is illustrative of the sulfo-estolide products used in the present technology that may be derived from, for example, linear unsaturated fatty acid feedstocks. It is understood that sultone hydrolyzed products and structures of a comparable nature may be derived from branched and/or substituted unsaturated fatty acids or mixtures of linear and branched and/or substituted unsaturated fatty acids.

Additional sulfo-estolide compositions may be produced from fatty acid feedstocks comprising polyunsaturated fatty acids, where A¹ and A² may be independently selected from alkyl diradicals that are: a) saturated; b) unsaturated; c) unsaturated and substituted with a sulfonate group; d) substituted with a hydroxyl group and a sulfonate group; e) substituted with a ester group and a sulfonate group (i.e., a sulfo-estolide).

In another embodiment of the present technology, the sulfo-estolide compositions are comprised of carboxylic esters, or are reported in an ester analysis as carboxylic esters. Although it is contemplated that at least some of these carboxylic esters are sulfo-estolides, the presently described technology is not limited by the accuracy of this belief, for example the compositions may contain carboxylic esters wherein X and Y within one or more repeating units, in general Formula 1, are both H (i.e., a hydrogen atom).

In another embodiment of the present technology, the sulfo-estolide compositions are comprised of sulfo-estolides of the general Formula 1 and a non-sulfonated estolide which comprises, for example, two or more fatty acid chains that does not contain a sulfonate group.

### Definitions

The term "sulfo-estolide" ("SE") is used herein to describe general Formula 1. The term "partially hydrolyzed sulfo-estolide" ("PHSE") describes compositions of general Formula 1 wherein the esters have been partially hydrolyzed (from between about 1% to about 95%). The term "hydrolyzed sulfo-estolide" ("HSE") describes compositions of general Formula 1 wherein the esters have been fully hydrolyzed (greater than about 95%, for example).

The term "sultone hydrolyzed product" ("SHP") is used herein to describe salts of sulfo-estolides that are produced from one or more feedstocks comprising unsaturated fatty acids by a process comprising the steps of sulfonation with SO₃, neutralization, and hydrolysis of sultones. The neutralization and hydrolysis are conducted at a level of caustic addition that maintains the pH in the range from about 4 to about 10.

The resulting product contains carboxylic acid esters at a level that corresponds to about 5 to about 95 mol%, alternatively about 20 mol% to about 60 mol%, alternatively about 20 mol% to about 45 mol%, alternatively about 30 mol% to about 45 mol% of the total carboxylic functionality in the composition. Although not wanting to be bound by any particular theory, it is believed that none or few of the esters (whether they are sulfo-estolides or not) are hydrolyzed in the process of making SHP. By processing at a low temperature and neutralizing the acid as it leaves the sulfonator as quickly as possible, it is further believed that lower ester levels will be obtained. Through improvement and modification of process conditions for the production of esters, it is contemplated that products of the present technology that have higher ester content will be obtained. For example, it is believed that the ester content may be obtained at lower and/or higher levels through the selection of the molar ratio of SO₃ to alkene functionality used in the sulfonation step, or alternatively or in addition, through the selection of the amount of monounsaturated and/or polyunsaturated fatty acids comprising the unsaturated fatty acid feedstock.

The term "ester hydrolyzed product" ("EHP") is used herein to describe one or more sulfonate compositions of the present technology that is produced from unsaturated fatty acids by sulfonation with SO₃ to produce sulfo-estolide and subsequent hydrolysis of greater than about 95% of the carboxylic esters. For example, the resulting product may have a carboxylic ester content that corresponds to less than about 5 mol %, alternatively less than about 2 mol%, alternatively less than 1 mol% of the total carboxylic functionality in the composition.

The term "partially ester hydrolyzed products" ("PEHP") is used herein to describe salts of sulfo-estolides used in the present technology that are produced from unsaturated fatty acids by sulfonation with SO₃ and hydrolysis of a portion of the carboxylic esters. The molar percentage of hydrolysis of carboxylic esters that is realized is from about 1% to about 95%, alternatively from about 5% to about 90%, alternatively from about 10% to about 90%, alternatively from about 20% to about 90%.

As defined herein, the term "sulfo-estolide surfactant" is meant to refer to a variety of surfactant compositions of the present technology as described, for example, by general Formula 1. This includes, for example, surfactant compositions of SE, HSE, PHSE, SHP, EHP, and PEHP.

As defined herein, the term "free alkalinity" is meant to refer to the total amount of carboxylate anion and hydroxide present in a composition, as may be measured by, for example, potentiometric titration of an aqueous solution with aqueous strong acid, for example HCl, to an endpoint of about pH 3 to about pH 4.5, or alternatively to bromophenol blue endpoint

As defined herein, the term "free caustic" is meant to refer to the total amount of excess strong alkalinity present in a composition, as may be measured by, for example, potentiometric titration of an aqueous solution with aqueous strong acid, for example HCl, to an endpoint of about pH 9 to about pH 11.

A "repeating unit" means one instance of the subject matter enclosed by brackets in a formula. For example, if n = 15 for a given molecule according to general Formula 1, the molecule has 15 instances of the bracketed structure. Each instance of the bracketed structure can be identical to or different from other instances of the bracketed structure. For example, the Y moiety in general Formula 1 can be H in one repeating unit and -SO₃⁻Z in another repeating unit of the same molecule.

### Making SE or Other Carboxylic Esters

A suitable starting material for the process of making one or more components and/or formulations of the present technology, for example, is a fatty acid (fatty carboxylic acid). Fatty acids that may be suitable for use in the present technology include, but are not limited to linear unsaturated fatty acids of 8 to 24 carbons, branched unsaturated fatty acids of 8 to 24 carbons, or mixtures thereof. Unsaturated fatty acids provided from commercial sources containing both saturated and unsaturated fatty acids are suitable for use in the practice of the present technology. Mixtures of saturated fatty acids and unsaturated fatty acids are also contemplated. In a non-limiting example, fatty acid mixtures that are rich in oleic acid (cis-9-octadecenoic acid) are suitable feedstocks. Other unsaturated fatty acids, for example but not limited to, trans-octadecenoic acids or palmitoleic acid may also be employed in the presently described technology.

Suitable feedstocks may be derived from vegetable and/or animal sources, including but not limited to fatty acids and fatty acid mixtures derived from, for example, canola oil, corn oil, cottonseed oil, linseed oil, olive oil, palm oil, peanut oil, rapeseed oil, safflower oil, sesame oil, soybean oil, sunflower oil, tall oil, tung oil, lard, poultry fat, BFT (bleachable fancy tallow), edible tallow, coconut oil, cuphea oil, yellow grease and combinations of these. Also contemplated are genetically modified or engineered oils that include, but are not limited to high oleic sunflower or soybean oil. In some embodiments, the preferred unsaturated fatty acid feedstocks may contain reduced levels of polyunsaturated fatty acids, for example, less than about 15%, alternatively less than about 10%, alternatively less than about 5% on a total weight basis. In some additional embodiments, the fatty acid feedstocks may be obtained by the partial hydrogenation of unsaturated triglycerides, for example, soybean oil followed by hydrolysis of the oil to afford fatty acids that are enriched in monounsaturated fatty acids and depleted in polyunsaturated fatty acids. The above-noted triglycerides optionally hydrogenated, can also be used as feedstocks, alone or in combination with fatty acids. Still further, in some embodiments of the presently described technology, suitable feedstocks may include those that contain appreciable amounts of saturated fatty acids, for example, up to about 80%, alternatively up to about 50%, alternatively up to about 30%, alternatively up to about 20% saturated fatty acid by weight. Alternatively, the feedstocks may be enriched in mono-unsaturated fatty acids, for example, via distillation; however, undistilled feedstocks are preferred due to lower cost.

In certain embodiments, a chain termination agent can be included in the reaction to reduce or prevent the formulation of products of general Formula 1 in which n is greater than one. The chain termination agent can be, for example, a saturated or unsaturated, substituted or unsubstituted, aliphatic or aromatic carboxylic acid having from 7 to 22 carbon atoms, or a combination of any two or more of these. The contemplated characteristic of a chain termination agent preferred for the present purpose is that it can form an ester. One class of preferred chain termination agents is a saturated fatty acid having from 8 to 22 carbon atoms, optionally from 8 to 14 carbon atoms, optionally 8, 10, or 12 carbon atoms or mixtures of these fatty acid species.

The compounds of general Formula 1 and related compounds (for example, where n = 0) can be made, for example, by: a) SO₃ sulfonation of a fatty acid, for example oleic acid; b) neutralization with aqueous caustic to afford a sulfonate salt solution with a pH in the range of about 4 to about 10; or c) hydrolysis of the resulting sultones, maintaining the reaction mixture at a pH of about 4 to about 10, alternatively at a pH of about 6 to about 8, alternatively at a pH of about 7. Sulfonation can be carried out, for example, using a falling film SO₃ process.

Alternatively, the compounds of general Formula 1 and related compounds (for example, where Z = H and W = H) can be made, for example, by falling film SO₃ sulfonation of a fatty acid, for example oleic acid where the process temperature of the sulfonation is sufficient, for example greater than about 20° C, to result in the formation of carboxylic esters.

Continuous SO₃ sulfonation processes, including those that utilize falling film reactors such as those described in Kirk-Othmer Encyclopedia of Chemical Technology, 5th ed., Vol. 23, Wiley-Interscience, Hoboken, NJ: 2007, entry entitled "Sulfonation and Sulfation", pp. 513-562, are suitable for conducting the sulfonation of feedstocks comprising unsaturated fatty acids in accordance with the practice of the presently described technology. For example, a monotube concentric reactor, annular film reactor, or multitube film reactor can be used to contact an unsaturated fatty acid feedstock, for example oleic acid, with a gaseous stream of SO₃ that is diluted with dry air. The molar ratio of SO₃ to alkene functionality in the fatty acid feedstock may be from about 0.3 to about 1.3, alternatively from about 0.5 to about 1.2, alternatively from about 0.8 to about 1.1, or alternatively from about 0.9 to about 1.0.

In some embodiments, a preferred ratio, for example, is less than about 0.8 so as to minimize color formation. Well, this could be the case for when less color is desired, which can happen for an LDL, but it is believed that as one decreases the degree of sulfonation, the desirable viscosity reducing properties would be diminished. The fatty acid feedstock is provided to the reactor at a temperature above the melting point of the feedstock, i.e. the feedstock is provided as a liquid. The sulfonation is conducted such that the reaction mass is maintained as a mobile liquid throughout the course of reaction. Preferably, a means of cooling the reaction mixture during the course of contact between the feedstock stream and the gaseous SO₃ stream is provided so that the sulfonic acid product is produced from the reactor at a temperature of from about 10° C to about 80° C, alternatively from about 20° C to about 60° C, or alternatively from about 30° C to about 60° C.

Sulfonated unsaturated fatty acid salt and sulfonated hydroxy fatty acid salt products include, for example, those sold in Europe as Polystep® OPA by Stepan Co. (Northfield IL), and as Lankropol OPA and Lankropol OPA-V by Akzo Nobel (Amsterdam), and in the United States as Calsoft® OS-45S by Pilot Chemical (Santa Fe Springs CA).

SE is produced from the sulfonation step and comprises carboxylic esters, provided that the reaction conditions are sufficient, for example, a high enough temperature of the acid stream, to promote carboxylic ester formation. While not limiting the scope of the presently described technology, the temperature at which carboxylic ester formation may occur is greater than about 10° C, alternatively greater than about 20° C, or alternatively greater than about 30° C. The sulfonic acid products may further comprise sulfonic acid esters, including but not limited to cyclic esters, i.e., sultones.

The process of making a sulfo-estolide mixture, including the methods of hydrolyzing suftones, hydrolyzing carboxylic esters and steps of bleaching the sulfono-estolides used in the present technology is described in PCT Application WO 2010/085247.

### Product Descriptions

Again not wanting to be bound by any particular theory, the compositions of the present technology as described by general Formula 1, are believed to be comprised of complex mixtures of compounds that are monomeric, dimeric, and higher-order oligomeric species in terms of the number of originating fatty acid chains. The oligomerization in these mixtures is via the formation of ester linkages. Branched oligomers are also envisaged.

The sulfo-estolide functional group corresponds structurally to the condensation of the hydroxyl group of an internal hydroxy sulfonate of fatty acid with the carboxylic acid group of a second fatty acid chain, where the second fatty acid chain may be, but is not necessarily limited to: a) an unsaturated or saturated fatty acid; b) an internal hydroxy sulfonate of fatty acid; c) an internal alkene sulfonate or corresponding cyclic anhydride (i.e., sultone) of fatty acid; or d) an internal mono- or poly-sulfo-estolide of two or more fatty acids (i.e., trimer, tetramer, etc.). The position of the sulfonate group along the back bone of the fatty acid chains is dictated by the location of the double bond in the starting material (9-octadecenoic acid for example) and the "direction" in which SO₃ adds across the double bond (thus, 9- and 10- sulfonate positions from oleic acid).

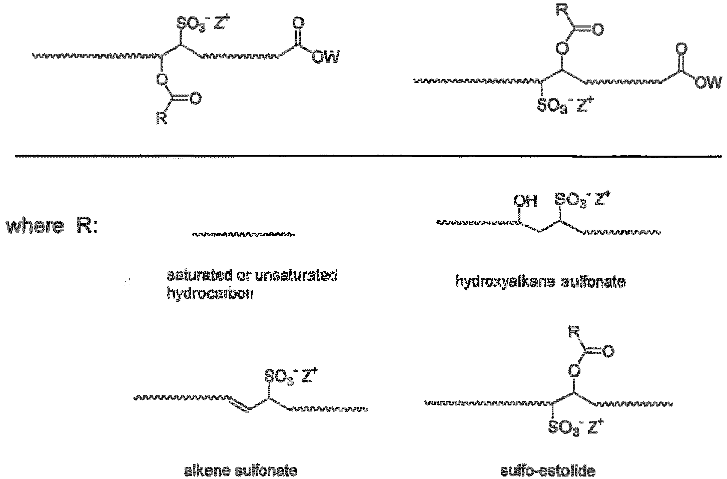

Non-ester-containing monomeric components made by this process are believed to comprise, in part, specific internal hydroxy sulfonates of fatty acid. For example, with 9-octadecenoic acid, the sulfonate groups are believed to be attached to the 9-position and alternatively the 10-position of the fatty acid. Examples are shown below.

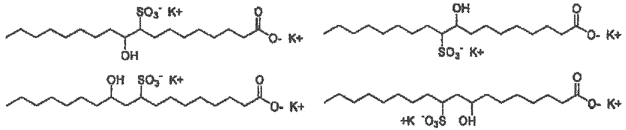

The monomeric components are further believed to comprise, in part, specific internal alkene sulfonates of fatty acid. These components may comprise cis- and/or trans-double bonds. It is also possible that compounds described herein are present where the unsaturation is at the position of the sulfonate group (i.e., vinylic sulfonates). Examples are shown below.

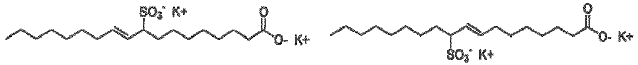

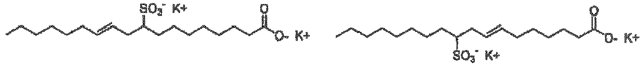

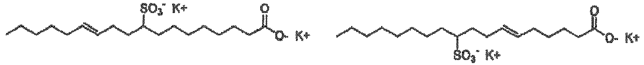

The monomeric components may further comprise disulfonated species, unsaturated fatty acids, and saturated fatty acids.

EHP is sometimes used herein as a designation for sulfonated products that have been subjected to complete hydrolysis of sulfo-estolide functionality. Such hydrolysis can be accomplished by, for example, treatment of SHP with excess base under high pH conditions (for example greater than about 11) at elevated temperatures (for example about 85° C to about 100° C). EHP is believed to comprise a mixture of hydroxyalkane sulfonates and alkene sulfonates of comparable structure to the monomeric components of sulfo-estolide compositions, though not necessarily in comparable ratios. Such mixtures are comparable to the compositions, for example, of sulfonated unsaturated fatty acids that are described, for example, in T. W. Sauls and W. H. C. Rueggeberg, Journal of the American Oil Chemists Society (JAOCS),Volume 33, Number 9, September, 1956, pp 383-389. It can be appreciated that partially ester hydrolyzed products (PEHP) will be comprised of elevated amounts of monomeric hydroxyalkane sulfonates and alkene sulfonates while maintaining some level of sulfo-estolide functionality.

### Formulations and Applications of SE

The compositions as described in the present technology may be used in formulations including, for example, light duty liquid detergents. The formulations of the present technology can be used in all delivery processes such as, but not limited to, Ready-To-Use, dilutable, wipes, single use, etc. These formulations, in some embodiments are stable with enzymes, peroxide, hypochlorite bleach, and other bleaching agents.

The formulations of the present technology may also be included in compositions including, for example, a cleaning adjunct Common cleaning adjuncts are identified in, for example, U.S. Patent 7,326,675, col. 12, and PCT Publ. WO 99/05242 (Pages 29-56). Such cleaning adjuncts are identified as including, but not limited to bleaches, bleach activators, suds boosters, dispersant polymers (e.g., from BASF Corp. or Rohm & Haas) such as the ACUSOL 400 series based on acrylic acid, color speckles, silvercare, anti-tarnish and/or anti-corrosion agents, pigments, dyes, fillers, germicides, hydrotropes, anti-oxidants, enzyme stabilizing agents, pro-perfumes, carriers, processing aids, solvents, dye transfer inhibiting agents, brighteners, structure elasticizing agents, fabric softeners, anti-abrasion agents, and other fabric care agents, surface and skin care agents. Suitable examples of such other cleaning adjuncts and levels of use in the practice of the present technology are described, for example, in U.S. Pat. Nos. 5,576,282, 6,306,812 B1 and 6,326,348 B1 and PCT Publ. WO 99/05242.

### General Considerations for Light Duty Liquid Detergents (LDL)

Desirable attributes of the present technology include an ability of being in liquid form at room temperature; an ability to formulate in cold-mix applications; an ability to perform as good as or better than existing conventional surfactants or formulations containing such conventional surfactants with respect to foaming level and soil removal, as well as other properties as described herein.

For household, industrial and institutional cleaning products, both surfactants and solvents are important additional ingredients. Desirable attributes for such products include, for example, the ability to emulsify, suspend or penetrate greasy or oily soils and suspend or disperse particulates, in order to clean surfaces; and then prevent the soils, grease, or particulates from re-depositing on the newly cleaned surfaces; and continue to produce foam in the presence of the soils being cleaned. In order to optimize these attributes, it is desirable to produce LDLs that contain moderate to high levels of surfactants (e.g. from about 20% to about 100% surfactant, alternatively from about 25% to about 80% surfactant, alternatively from about 25% to about 70% surfactant). However, prior to the present technology, LDLs containing such high levels of surfactants were gels, not liquids, at room temperature. These gels were not workable, not easily dispensed or poured, at room temperature. Formulators would typically add solubilizing solvents like ethanol to convert the gels to liquids, but materials like this add flammability concerns and have minimal contribution to cleaning and foaming. Surprisingly, the components and formulations of the present technology demonstrate that the addition of at least one sulfo-estolide surfactant, having the general Formula 1, to LDL formulations decreases the viscosity for such formulations into the workable liquid range at room temperature (200cps to 6000cps). Further, the sulfo-estolide containing LDL formulations of the present technology containing one ore more sulfo-estolides as described herein maintain the high foaming and optimized cleaning attributes described above, among others.

Formulations are contemplated having a viscosity of about 5 cPs to about 2000 cPs, measured at 25° C using a Brookfield Viscometer model LV, with spindle 2, 3 or 4 at speeds ranging from about 12 rpm to about 50 rpm. LDL formulations of the present technology containing at least one sulfo-estolide surfactant having the general Formula 1 have been surprisingly found to have lower viscosity than comparable formulations lacking such surfactants. Since these compositions function as viscosity reducers, they are very useful for making the contemplated highly concentrated, (e.g. greater than about 20% surfactant active, and even beyond 40% active) LDL detergent formulations. Liquid compositions greater than 40% active would be very useful for performance and economy, but have heretofore been unattainable except for the use of large quantities of undesirable solubilizing alcohols as described above.

Various formulations of the present technology exhibit viscosities of from about 100 cps to about 10,000 cps; alternatively, from about 200 cps to about 6,000 cps, measured at 25° C using a Brookfield Viscometer model LV, with spindle 2, 3 or 4 at speeds ranging from about 12 rpm to about 50 rpm.

It is also desirable to have the ability to control the foaming of different household, industrial and institutional products depending on the desired end-use applications. For example, for one or more light duty liquid detergents of the present technology, it is desirable to have suitable foaming ability along with a viscosity that is workable (viscosity of 200cps to 6000cp measured at 25° C using a Brookfield Viscometer model LV, with spindle 2, 3 or 4 at speeds ranging from about 12 rpm to about 50 rpm) at room temperature.

It is also desirable to have the ability to produce "green" LDL formulations. Thus, the surfactants should be ultimately biodegradable, phosphate free, and non-toxic. To meet consumer perceptions and reduce the use of petrochemicals, a "green" formula may also advantageously be limited to the use of renewable hydrocarbons, such as vegetable or animal fats and oils, in the manufacture of one or more surfactant components. The presently used sulfoestolide surfactants are derived from plant and/or animal fats and oils and thereby address this challenge.

It is also desirable for the pH of LDL detergents to be in the range in which contact with hands and skin is acceptable while maintaining adequate foaming and cleaning properties. The presently described compositions achieve this need by possessing adequate soil removal and foaming properties at or around neutral pH. Sulfo-estolide surfactant containing LDL detergents of the present technology have pH values in the range of from 3 to 10; alternatively, from 4 to 9; and preferably from 6 to 8.

### Formulations

A wide variety of compositions can be made that include at least one sulfo-estolide surfactant or two or more sulfo-estolide surfactants, as described herein, with or without other ingredients as specified herein. Formulations are contemplated containing, for example, sulfo-estolide surfactants from between 0.1% to 70% by active weight; alternatively between about 0.1% to 50% by active weight; alternatively, between 0.1% to 35% by active weight; alternatively, between 1% to 30% by active weight based on the total actives ingredient weight of the composition.

The sulfo-estolide surfactants having the general Formula 1 described herein can be incorporated into, for example, various formulations and used as surfactants, emulsifiers, skin feel agents, film formers, rheological modifiers, solvents, release agents, lubrication agents, conditioners, dispersants, hydrotropes, etc. Such compositions can be used in end-use applications including, but not limited to, household and industrial and institutional cleaning products.

In alternative embodiments, sulfo-estolide surfactants having the general Formula 1 can be used to produce antimicrobial formulations. One or more sulfo-estolide based LDL antimicrobial compositions of the present technology can include from 0% to 10% by weight of a polyvalent metal ion chelant, alternatively from 0.1% to 10%, alternatively from 1% to 10%, alternatively from 1% to 5% by weight, and may additionally include any range or percentage there between, including, but not limited to, for example, increments of 0.1, 0.2, 0.3, 0.4, 0.5, 0.6, 0.7, 0.8, 0.9, 1.0. 2.5, 5 % and multiplied factors thereof. Further, the antimicrobial compositions can further include from 0% to 10% of an alkaline builder, alternatively from 0.1% to 10%, alternatively from1% to 10%, alternatively from 1% to 5% by weight, and may additionally include any range or percentage there between, including, but not limited to, for example, increasing or decreasing increments of 0.1, 0.2, 0.3, 0.4, 0.5, 0.6, 0.7, 0.8, 0.9 or 1.0% 2.5%, 5% and multiplied factors thereof such as 1.5x, 2.0x, 3.0x, 4.0x, 5.0x and 6.0X as desired to achieve higher concentrates. Suitable alkaline builders include, but are not limited to sodium carbonate, potassium pyrophosphate, sodium metasilicate, or combinations thereof. Further, such antimicrobial compositions may also include at least one additional component, for example dyes and fragrances, from 0% to 2% by weight, alternatively from 0.01% to 2%, alternatively from 0.1% to 2%, alternatively from 0.1% to 1% by weight, and including any percentage or range there between, including, but not limited to for example, alternatively from 0.1% to 10%, alternatively from 1% to 10%, alternatively from 1% to 5% by weight, and may additionally include any range or percentage there between, including, but not limited to, for example, increasing or decreasing increments of 0.1, 0.2, 0.3, 0.4, 0.5, 0.6, 0.7, 0.8, 0.9 or 1.0 %, 2.5%, 5% and multiplied factors (1.5x, 2.0x, 3.0x, 4.0x, 5.0x and 6.0x) thereof.

LDL antimicrobial components of the present technology can also include, but are not limited to triclosan, n-alkyl dimethyl benzyl ammonium chloride, dialkyl dimethyl ammonium chloride, didecyl dimethyl ammonium chloride, dioctyl dimethyl ammonium chloride, phenolics, iodophors, pine oil, methyl salicylate, morpholine, silver, copper, bromine, and quaternary ammonium compounds, derivatives thereof, and combinations thereof including, but not limited to, the polyquaternium series as is used in hand soap formulations, and 3,4,4'trichlorocarbanilide as disclosed in US 6,605,579.

Suitable antimicrobial agents can be found in McCutcheons' 2009 Functional Materials of North American Edition, Volume 2, 2009, pages 239-246, Suitable antimicrobial agents include, but are not limited to, Abiol, which is available from 3V Inc. (Brooklyn, NY); Phenobact, which is available from Alzo International, Inc. (Sayreville, NJ); Emercide 1199, which are available from Cognis Canada Corp. (Mississauga, ON); Bronidox 1160, which is available from Cognis Corporation Care Chemicals (Monheim, Germany); Custom D Urea, Custom DMDM, Custom I Urea, Custom Methyl Paraben, Custom PCMX, Custom PCMX 25%, Custom Propyl Paraben, Salicat K 727, Salicat K100, Salicat K145, Salicat MM, Saligerm G-2, Salinip, which are available from Custom Ingredients, Inc. (Chester, SC); Bioban BP-Pharma, Bioban BP-Plus, Bioban CS-1135, Bioban CS-1246, Bioban P-1487, Dowicil 75, Dowicil 200, Dowicil QK-20, Fuelsaver, Oxaban-A (78%), Oxaban-A (90%), Tris Nitro concentrate, Ucarcide, which are available from Dow Chemical Company (Wilmington, DE); Generic Propylene glycol, which is available from Huntsman Corporation Performance Products (The Woodlands, TX); Bronopol, Lexgard 688, Lexgard 690, Lexgard B, Lexgard GMC, Lexgard GMCY, Lexgard M, Lexgard MCA, Lexgard O, Lexgard P, Myacide SP, which are available from Inolex Chemical Co. Personal Care Application Group (Philadelphia, PA); Anthium Dioxide, which is available from International Dioxide, Inc. (North Kingstown, RI); Germaben II, II-E, Germall II, Germall 115, Germall Plus, LiquaPar Oil, LiquaPar Optima, LiquaPar PE, Liquid Germall Plus, Methyl Paraben, Propyl Paraben, Suttocide A, which are available from International Specialty Products/ISP (Wayne, NJ); Liposerve DU, Liposerve DUP, Liposerve IU, Liposerve MM, Liposerve PP, which are available from Lipo Chemicals, Inc. (Paterson, NJ); Dantogard, Dantogard 2000, Dantogard Plus, Dantogard Plus Liquid, Dantogard XL-1000, Dantoserve MS, Dantoserve SG, Geogard 111 A, Geogard 111 S, Geogard 221, Geogard 233 S, Geogard 234 S, Geogard 361, Geogard Ultra, Glycacil, Glycacil 2000, Glycacil SG, Glydant, Glydant 2000, Glydant Plus, Glydant Plus Liquid, Glydant XL-1000, which are available from Lonza Inc. (Allendale, NJ); Mackstat 2G, Mackstat OM, Mackstat SHG, Paragon, Paragon II, Paragon III, Paragon MEPB, Phenagon PDI, which are available from The McIntyre Group (Norwalk, CT); Merguard 1105, Merguard 1190, Merguard 1200, which are available from Nalco Company (Naperville, IL); Britesorb A 100, which is available from The PQ Corp (Malvern, PA); Generic Methylparaben NF, Generic Propylparaben NF, Generic Ethylparaben NF, Generic Butylparaben NF, which are available from RITA Corp. (Crystal Lake, IL); Kathon CG, Kathon CG II, Kathon CG/ICP, Kathon CG/ICP II, Kathon LX 1.5% Microbicide, Koralone B-119 Preservative, Koralone N-105, Kordek MLX, Lanodant DM, Neolone 950, Neolone CapG, Neolone DsP, Neolone M-10, Neolone MxP Preservative, Neolone PE Preservative, Rocima 550 Microbicide, Rocima 586, Rocima 607/ Microbicide, Rocima BT 2S, Rocima BT NV 2, which are available from Rohm and Haas Go./Consumer and Industrial Specialties (Philadelphia, PA); Vancide TH, which is available from R.T. Vanderbilt Co. Inc. (Norwalk, CT); PCMC, which is available from R.W. Greeff and Co., Inc./Howard Hall Div. (Stamford, CT); Sepicide HB, which is available from Seppic Inc. (Fairfield, NJ); Onamer M, Onyxide 200 Preservative, Stepanquat 50NF, Stepanquat 65NF, Stepanquat 200, Stepanquat 1010, Stepanquat 1010-80%, Stepanquat 1210-80%, which are available from Stepan Company (Northfield, IL); Grotan, Mergal 142, Mergal 174, Mergal 186, Mergal 192, Mergal 198, Mergal 364, Mergal 395, Mergal 586, Mergal 1000, Mergal K9N, Mergal K10N, Mergal K14, Mergal 1005, which are available from Troy Corporation (Florham Park, NJ), among others.

Optionally, the LDL detergent compositions of the present technology can include at least one additive as well. Suitable additives include, but are not limited to viscosity modifiers, electrolytes, thickeners, emollients, skin conditioning agents, emulsifier/suspending agents, solubilizing agents, fragrances, colors, dyes, herbal extracts, vitamins, builders, enzymes, pH adjusters, preservatives, antimicrobial agents, polymers, magnesium sulfate, derivatives thereof, combinations thereof, and other ingredients commonly known in the art as an additive.

Magnesium sulfate, builders, solubilizing agents and enzymes may be added to aid in cleansing ability, for example. Emollients (including, without limitation, vegetable oils, mineral oils, silicone oils, petrolatum, polyglycerol methyl esters, and esters), skin conditioning agents (such as glycerine and free fatty acid), vitamins and herbal extracts may be added to further improve conditioning performance. Fragrances, dyes, opacifying agents, and pearlescent agents may also be added to further enhance the appearance and smell of one or more of the finished LDL formulations of the present technology.

Suitable preservatives for use in the practice of the present technology include, but are not limited to acidics and phenolics, for example, benzoic acid and salts, sorbic acid and salts, propionic acid and salts, boric acid and salts, dehydroacetic acid, sulfurous and vanillic acids, Ottasept® (which is available from Ottawa Chemical Company (Toledo, OH)), Irgasan DP 300® (which is available from Geigy Chemical Corporation (Ardsley, NY)), phenol, cresol, chlorocresol, *o-*phenylphenol, chlorothymol, parabens, alkyl esters of parahydroxybenzoic acid, methyl, ethyl, propyl, benzyl, and butyl-*p*-hydroxybenzoates; mecurials, for example, thiomersal, phenylmercuric acetate and nitrate, nitromersol, sodium ethylmercurithiosalicylate; quaternary ammonium compounds, for example, benzalkonium chloride, cetylpyridinium chloride, benzethonium chloride, cetyltrimethyl ammonium bromide, Polyquad® (which is available from Alcon Research, Ltd. (Forth Worth, TX)); and other compounds, for example, methylchloroisothiazolinone, methylisothiazolinone, 2-methyl-4-isothiazolin-3-one, 1,2-benzisothiazolin-3-one, imidazolidinyl urea, 1,3-dimethylol-5,5-dimethylhydantoin, alcohols (ethyl alcohol), chlorobutanol, phenoxy-2-ethanol, benzyl alcohol, phenylethyl alcohol, chlorhexidine, polyaminopropyl biguanide, chloroform, 6-acetoxy-2,4-dimethyl-*m*-dioxane, 2,4,4-trichloro-2'-hydroxy-diphenylether, imidazolidinyl urea compound, bromo-2-nitropropanediol-1,3-bromo-5-nitrol-1,3-dioxane 2-methyl-4-isothiazoclin-3-one and 5 chloro derivative, 1-(3-Chloroallyl)-3,5,7-triazo-1-azoniaadamantane chloride (Dowicil 200)® (which is available from Dow Chemical Company (Midland, MI)), Bronopol® (which is available from Boots Company Limited (Nottingham, England)), Ucarcide® (which is available from Union Carbide Corporation (Danbury, CT)), Germal II®, Germal 115® (which are available from Produits Sanitaires Unique Inc. (La Pocatiere, QC), Glydant® (which is available from Lonza, Inc. (Fairlawn, NJ)), Mycide SP®, Kanthon CG®, Oxadine A®, Omadine® (which is available from Olin Corporation (New Haven, CT)), Phenoxetol® (which is available from Nipa Laboratories, Ltd. (Manchester, England)). Suitable preservatives for personal care products can be found in Preservatives for Cosmetics Manual, Second Edition, by David S. Steinbens, 2006.

Enzymes suitable for use in the practice of the present technology include proteases, amylases, and lipases.

Polymers suitable for use in the practice of the present technology include, for example, anionic polymers, acrylates, hydroxyethylcelluloses, zwitterionic polymers, gelatins, xanthan gums, polysaccharides, and polyethylene glycols.

Sulfo-estolide surfactant containing LDL detergents of the present technology that comprise from 1% to 99% of at least one carrier are also contemplated. As will be appreciated by at least those skilled in the art, a variety of carriers, vehicles, diluents, and the like are suitable for use in the practice of the present technology. Thus, it will also be appreciated that the terms "carrier", "vehicle", and "diluent" are to be considered non-exhaustive with respect to the present technology and in describing the various formulations, applications, compositions, et cetera thereof.

The sulfo-estolide containing LDL detergent compositions described herein are preferably in the form of non-emulsion liquids in which water is the principal carrier. Alternatively, although less preferred, other solvents such as alcohols may be utilized in combination with water. The level of water in a liquid cleaning composition is preferably from 10% to 99% by weight, alternatively from 20% to 50% by weight. Solvents that may be practiced in connection or conjunction with the present technology include, but are not limited to, 1,3-propanediol, propylene glycol, glycerol, ethanol, glycol ethers, derivatives thereof, combinations thereof, and others.

### Additional Surfactants and Foam Stabilizing Surfactants

The compositions of the present technology can contain additional surfactants and foam stabilizing surfactants, which can be anionic, cationic, nonionic, ampholytic (includes usage of the term amphoteric), amphoteric, zwitterionic, in nature or combinations thereof. Suitable co-surfactants for use in light duty liquid detergents are described, for example, in PCT Application WO 2010/085247.

Certain embodiments of the present technology contain additional surfactants in the amounts of from 2% to 70% by active weight; alternatively, from 5% to 45% by active weight; alternatively, from 10% to 30% by active weight based on the total actives ingredient weight of the composition.

Preferred additional surfactants for use in the present technology include, for example, Steol CS-270 (lauryl 2-mole average ether sulfonate), Steol CS-170 (lauryl 1-mole average ether sulfonate), Steol CS-330 (lauryl 3-mole average ether sulfonate), Bio-Soft EC-690 (alcohol ethoxylate), Bio-Soft D-40 (sodium alkylbenzenesulfonate), Bio-Soft S-101 (alkylbenzene sulfonic acid) neutralized with sodium, potassium, ammonium and/or magnesium, Bio-Terge AS-40 (sodium olefin sulfonate), and/or Stepanol WA-Extra K (sodium lauryl sulfate), all from the Stepan Company, Northfield Illinois. Any of the aforementioned anionic surfactants may be neutralized to form the sodium, potassium, ammonium or magnesium salts.

Certain embodiments of the present technology can contain foam stabilizing surfactants in amounts of from 0.5% to 15% by active weight; alternatively, from 3% to 10% by active weight; alternatively 5% by active weight based on the total actives ingredient weight of the composition.

Preferred foam stabilizing surfactants for use in the present technology can include Amphosol CA (cocoamidopropyl betaine), Ammonyx LMDO (lauryl myristal amidopropyl dimethyl amine oxide), Ammonyx LO (lauryl dimethyl amine oxide) all from the Stepan Company, Northfield Illinois, as well as Glucopon 600 (alkyl polyglucoside), and Glucopon 425 N (alkyl polyglucoside), both from the Cognis Company, Monheim Germany.

### Anionic Surfactants

"Anionic surfactants" are defined here as amphiphilic molecules with an average molecular weight of less than about 10,000, comprising one or more functional groups that exhibit a net anionic charge when in aqueous solution at the normal wash pH, which can be a pH between about 5 to about 11. The anionic surfactant used in the present technology can be any anionic surfactant that is substantially water soluble. "Water soluble" surfactants are, unless otherwise noted, include surfactants which are soluble or dispersible to at least the extent of 0.01% by weight in distilled water at 25° C.

Another important class of anionic compounds is the water soluble salts, particularly the alkali metal salts, of organic sulfur reaction products having in their molecular structure an alkyl radical containing from 6 to 24 carbon atoms and a radical selected from the group consisting of sulfonic and sulfuric acid ester radicals.

Additional anionic surfactants for use in the present technology include, but are not limited to, sulfoacetates, olefin sulfonates, alkyl benzene sulfonates, alkyl sulfosuccinates, alkyl sulfomethylsuccinates, derivatives thereof, combinations thereof, among others.

### Cationic Surfactants

Cationic surfactants contemplated for use in the present technology include, for example, ditallow dimethylammonium chloride (DTDMAC), fatty alkanolamides (FAA), and quatemized diesters of trialkanolamines and fatty acids. The proportions of cationic surfactants used in one or more a formulations of the present technology can range, for example, from 0.1% to 20%, more preferably between 1% to 10%, even more preferably between 1% to 5%. See also P&G US patent 5,929,022; column 6, 2nd paragraph through column 7, 1st paragraph.

### Nonionic Surfactants

Examples of suitable nonionic surfactants for use in the practice of the present technology include alkyl polyglucosides ("APGs"), alcohol ethoxylates, nonylphenol ethoxylates, among others. The nonionic surfactant may be used in an amount of from 1% to 90%, more preferably from 1% to 40% and most preferably between 1% to 32% of an LDL detergent formulation of the present technology. Other suitable nonionic surfactants are described in P&G patent 5,929,022; column 4, 2nd paragraph through column 6, end of 1^{st} paragraph.

Preferred additional nonionic surfactants and foam stabilizing nonionic surfactants for use in the present technology include, but are not limited to alcohol ethoxylates, alkyl polyglucosides, alkyl ethanolamides, and alkyl esters.

### Ampholytic Surfactants

Ampholytic (includes usage of the term amphoteric) synthetic detergents can be broadly described as derivatives of aliphatic or aliphatic derivatives of heterocyclic secondary and tertiary amines, in which the aliphatic radical may be straight chain or branched and where one of the aliphatic substituents contains from 8 to 18 carbon atoms and at least one contains an anionic water-solubilizing group, e.g., carboxy, sulfo, sulfato, phosphato, or phosphono (see U.S. Patent No. 3,664,961, which provides specific examples of ampholytic surfactants from col. 6, line 60, to col. 7, line 53). Examples of suitable ampholytic surfactants include, for example, fatty amine oxides and fatty amidopropylamine oxides. At least one suitable example is cocoamidopropyl betaine (CAPB) also known as coco betaine. Ampholytic surfactants can be used at, for example, a level from 1% to 50%, more preferably from 1% to 10%, even more preferably between 1% to 5% of the formulation, by actives weight percent.

Some preferred foam stabilizing ampholytic surfactants for use in the practice of the present technology can include, but are not limited to amine oxides, amidopropyl amine oxides, betaines, amidopropyl betaines, sulfobetaines, hydroxysultaines, amphoacetates, amphopropionates, alkyl amines, organic diamines, derivatives thereof, or combinations thereof, among others.

### Zwitterionic Surfactants

Suitable zwitterionic synthetic surfactants for use in the practice of the present technology can be broadly described as derivatives of aliphatic quaternary ammonium and phosphonium or tertiary sulfonium compounds, in which the cationic atom may be part of a heterocyclic ring, and in which the aliphatic radical may be straight chain or branched, and where one of the aliphatic substituents contains from about 3 to 18 carbon atoms, and at least one aliphatic substituent contains an anionic water-solubilizing group, e.g., carboxy, sulfo, sulfato, phosphato, or phosphono. (see *e.g.,* U.S. Patent No. 3,664,961, which provides various examples of zwitterionic surfactants from col. 7, line 65, to col. 8, line 75). Zwitterionic surfactants can be used in various formulations of the present technology from 1% to 50%, more preferably from 1% to 10%, even more preferably from 1% to 5% by actives weight percent of the present formulations.

### Mixtures of Surfactants

Mixtures of any two or more individually contemplated surfactants, whether of the same type or different types, are envisaged.

### Builders

In some embodiments, the LDL detergent formulations of the present technology containing sulfo-estolide surfactants include at least one builder. Preferably, suitable phosphate-free builders known in the art are used. Builders included compositions comprising a mixture of sodium carbonate and/or sodium citrate and low molecular weight polyacrylic polymer, such as a polyacrylate organic and/or inorganic detergent builders, and the like. Other builder salts for use in the practice of the present technology including, but not limited to gluconates, phosphonates, nitriloacetic acid salts, combinations thereof, and derivatives thereof can be mixed with, for example, sodium bicarbonate and/or sodium citrate. Additional embodiments of the present technology are practiced with citric acid and/or citrate salt of a metal ion as the builder.

### EXAMPLES

The compositions and processes described here, and ways to make and use them are illustrated by the following examples. Examples stated in the present or future tense are not represented as having been carried out. Examples to the methods of producing and testing sulfo-estolides as described herein are disclosed in PCT Application WO 2010/085247,

### Examples 1-26.

### EXAMPLE 1: COMPARISON OF SURFACE ACTIVITES

The surface activities of SE were compared with other commonly used anionic surfactants, STEOL® CS-230 (Sodium Laureth Sulfate, 2EO), STEOL® CS-330 (Sodium Laureth Sulfate, 3EO), STEPANOL® WA-WXTRA (Sodium Lauryl Sulfate), all available from Stepan Company, Northfield, IL. The surface activity was measured using Kruss K12 tensiometer at 25°C in DI water. The results can be found in Table 1. The critical micelle concentration (CMC) and the surface tension at CMC are important properties for a surfactant. CMC indicates the minimum concentration of a surfactant that forms aggregates. The surfactant with lower CMC is more effective to emulsify or remove oil. The surface tension indicates how efficient a surfactant can reduce the surface energy of water. Lower surface tension is favorable for wetting and cleansing. The results showed that SE is an effective surfactant.

**Table 1**

| | CMC (mg/L) | Surface Tension @CMC (mN/m) |
|---|---|---|
| SE | 36.1 | 34.5 |
| STEPANOL WA-EXTRA (SLS) | 184.8 | 26.3 |
| STEOL CS-230 (SLES-2) | 171 | 25 |
| STEOL CS-330 (SLES-3) | 75 | 30 |

### EXAMPLE 2: LIGHT DUTY LIQUID DETERGENT COMPOSITION OF THE PRESENT TECHNOLOGY

Table 2 presents light duty liquid laundry detergent formulas. Formula B includes a sulfonated-estolide surfactant (SE) as described herein while Formula A does not. SE is a sulfonated estolide potassium salt produced from 100% Oleic acid feed stock. The SE product was the result of neutralization, hydrolysis, and bleaching (using 1.1% by weight of 50% H₂O₂ per acid flow). The final product consisted of 71.37% solids at a pH of 5.02 with a %K₂SO₄ of 2.41. The formulations also included sodium lauryl sulfate (STEPANOL WA-EXTRA K, available from Stepan Company, Northfield IL), cocoamidopropyl betaine (Amphosol CA, also available from Stepan Company) and deionized water.

The feedstock used in this example had an equivalent weight of about 275.06 and was comprised of about 78% C-18:1, about 12% C-18:2, and about 9% saturated fatty acids. The feedstock was sulfonated on a falling film reactor at a rate of about 58.65 kg (129.3 lbs) per hour using a molar ratio of SO₃ to alkene functionality of about 0.95. The SE sulfonic acid was continuously neutralized in a loop reactor with concurrent addition of about 22.3 kg (49.1 lbs) per hour of 45% aqueous KOH and about 17.2 kg (37.9 lbs) per hour of water. The temperature of the reaction mixture in the loop reactor was about 80°C. Neutralized SE solution was continuously fed from the loop reactor to an in-line mixer, where about 1.18 kg (2.61 lbs) per hour of 50% aqueous hydrogen peroxide was homogenized into the solution, which was about pH 5.8. This reaction mixture was then fed to a stirred tank reactor. After collecting about 227 liters (60 gallons) of reaction mixture, concurrent sultone hydrolysis and bleaching were continued at about 80°C for about 4 additional hours. At the end of this 4 hour hydrolysis and bleaching period about 7.48 kg (16.5 lbs) of 38% sodium bisulfite solution was added to the reaction mixture to reduce the residual peroxide in solution from about 0.25 % (wt/wt) active peroxide down to about 0.02% (wt/wt) active peroxide. The SHP produced from this reaction was at a pH of about 5.0, was comprised of about 69.8% solids and about 0.017 % (wt/wt) active peroxide, and had a Klett color at 1 percent solids concentration of 51. The EHP was analyzed by titration with aqueous HCl and was found to comprise about 40.8 mol percent of the carboxylic ester.

For each component, "% Active RM" indicates the percents of active material in the feedstock, "Formula % Active" indicates the weight percent of the active material in the liquid detergent formulation, and "Wt. Needed" and "Wt. Added" (both in grams) indicate the calculated and actually weighed amounts added to a formulation having a total weight of 100.00 grams. Each of these formulations are intended to be liquid detergent formulas and it is contemplated that additional optional components may be added.

**Table 2**

| **Formula A** | **No SE** | | | | | |
|---|---|---|---|---|---|---|
| **Component** | **Lot** # | **order** | **% Active RM** | **Formula % Active** | **Wt. Needed (gms)** | **Wt. Added** |
| DI Water | NA | 1 | 100.00 | - | 17.06 | 17.08 |
| SE | NA | 2 | 68.00 | 0.00 | 0.00 | 0.00 |
| Stepanol WA-Extra K | 7297969 | 3 | 30.00 | 20.00 | 66.67 | 66.68 |
| Amphosol CA | 7036625 | 4 | 30.73 | 5.00 | 16.27 | 16.23 |
| | | | | Total | 100.00 | |
| | | | | | | |

| **Formula B** | **With SE** | | | | | |
|---|---|---|---|---|---|---|
| **Component** | **Lot #** | **order** | % **Active RM** | **Formula** % **Active** | **Wt. Needed (gms)** | **Wt. Added** |
| DI Water | NA | 1 | 100.00 | - | 9.71 | 9.68 |
| SE | NA | 2 | 68.00 | 5.00 | 7.35 | 7.39 |
| Stepanol WA-Extra K | 7297969 | 3 | 30.00 | 20.00 | 66.67 | 66.65 |
| Amphosol CA | 7036625 | 4 | 30.73 | 5.00 | 16.27 | 16.28 |
| | | | | Total | 100.00 | |

The viscosity of the present formulations was measured at a temperature of 25°C with a Brookfield model RVT viscometer, with spindle #3 at 20 rpm. The viscosity of the Formula A without SE was 29,440 cps and was a clear viscous gel. The viscosity of Formula B containing SE was 6,425 cps and was a viscous clear liquid. Thus, addition of the SE described herein provides a 4-fold decrease in the viscosity of the formulation, allowing a previously unusable gel formulation to be usable as a pourable light duty liquid detergent.

The ability of the formulations (A and B) to foam was tested using a foam mileage procedure using Crisco vegetable shortening. For comparison, a commercially available light duty liquid detergent (Ultra Palmolive) was also tested. A 0.1% solution of the LDL is prepared in 500 grams total using 140ppm hardness tap water initially at 50 degrees Centigrade. This wash bath is agitated with a KitchenAid mixer at a setting of 6, producing copious initial foam. Crisco shortening, which serves as the soil in this procedure, is titrated into the wash solution at a rate of no more than 0.5 grams per minute with a syringe. As the soil is introduced, the foam eventually collapses. The amount of Crisco tolerated prior to foam collapse is the foam mileage for the formula. This simulates soil being introduced form the washing of dirty plates, and measures how many plates could be washed before the foam is gone. The results of the foam mileage test are shown in Table 3, wherein the addition of the SE increases the foam mileage of the formulation.

**Table 3**

| **Product** | **% Solution** | **Rep** | **Run** # | **Wt (start)** | **Wt (end)** | **Soll wt** | **Average** |
|---|---|---|---|---|---|---|---|
| **Formula A** | 0.1000 | 1 | 3 | 12.23 | 9.81 | 2.42 | 2.35 |
| | | 2 | 4 | 17.80 | 15.53 | 2.27 | |
| **Formula B** | 0.1000 | 1 | 1 | 17.81 | 14.98 | 2.83 | 2.79 |
| | | 2 | 2 | 14.98 | 12.23 | 2.75 | |
| **Ultra Palmolive** | 0.1000 | 1 | 5 | 15.53 | 12.32 | 3.21 | 3.15 |
| | | 2 | 6 | 12.32 | 9.24 | 3.06 | |

### EXAMPLE 3: EXEMPLARY FORMULATIONS OF LIGHT DUTY LIQUID DETERGENTS THAT CONTAIN SULFO-ESTLIDE SURFACTANTS

Table 4, 5 and 6 provide exemplary formulations of liquid light duty detergents. These formulations provide pourable liquid formulations of light duty detergents.

| **Formulation 1** | **High Active LDL Concentrate - "Ultra Ultra"** | | | |
|---|---|---|---|---|
| **Ingredient** | **Function** | **Wt as is** | **Raw Actives %** | **Formula Active %** |
| Stool CS-270 | Primary | 41.18 | 68 | 28.0 |
| SE | Co-Primary | 41.18 | 68 | 28.0 |
| Ammonyx LMDO | Secondary | 17.64 | 33 | **5.8** |
| | Total | 100 | | 61.8 |
| This composition contains about double the "typical" level of surfactant actives, yet is a flowable viscous liquid | | | | |

In Formulation 1, Stepanol WA-Extra or other olefin sulfonate may be used as the primary surfactant. Further, Ammonyx LO, betaine or sulfobetaine or other amine oxide or amidopropyl amine oxide, or alkanolamide may be used as a secondary surfactant.

**Table 5**

| **Formulation 2** | **High Active LDL Concentrate - "Ultra Ultra"** | | | |
|---|---|---|---|---|
| **Ingredient** | **Function** | **Wt as is** | **Raw Actives %** | **Formula Active %** |
| Steol CS-270 | Primary | 41.18 | 68 | 28.0 |
| SE | Co-Primary | 41.18 | 68 | 28.0 |
| Ammonyx LO | Secondary | 9.84 | 30 | 3.0 |
| Glucopon 600 | Secondary | 7.8 | 55 | 4.3 |
| | Total | 100 | | 63.2 |
| This composition contains about double the level of surfactant actives, yet is a flowable viscous liquid | | | | |

In Formulation 2, Stepanol WA-Extra or other olefin sulfonate may be used as the primary surfactant. Further, Ammonyx LMDO, betaine or sulfobetaine or other amine oxide or amidopropyl amine oxide, or alkanolamide may be used as a secondary surfactant. Also, any other Glucopon may be substituted for the Glucopon 600 secondary surfactant.

**Table 6**

| **Formulation 3** | **High Active LDL Concentrate** - **"Ultra Ultra" Antimicrobial** | | | |
|---|---|---|---|---|
| **Ingredient** | **Function** | **Wt as is** | **Raw Actives %** | **Formula Active %** |
| Bio-Soft EC-690 | Primary | 60 | 90 | 54.0 |
| SE | Co-Primary | 20 | 68 | 13.6 |
| Ammonyx LMDO | Secondary | 9.7 | 30 | 2.9 |
| Glucopon 425 N | Secondary | 10 | 55 | 5.5 |
| Triclosan | Antimicrobial | 0.3 | 100 | 0.3 |
| | Total | 100 | | 76.3 |
| This composition contains about triple the level of surfactant actives, yet is a flowable liquid | | | | |

Table 6 demonstrates exemplar formulations of an antimicrobial formulation of the present technology.

In Formulation 3, a blended primary nonionic surfactant or Glucopon may be substituted for Bio-Soft EC-690 in the primary/secondary surfactant system. Further, Ammonyx LO, betaine or sulfobetaine or other amine oxide or amidopropyl amine oxide, or alkanolamide may be used as a secondary surfactant. Also, any other Glucopon may be substituted for the Glucopon 425N secondary surfactant.

### EXAMPLE 4: EXEMPLAR FORMULATIONS

The following prophetic formulas, in Table 7, are intended to cover light duty liquid detergents. These formulations are not intended to be limiting in any way - optional ingredients described herein regarding the present technology can be added in the proportions described. In each case, these are intended to be liquid detergent formulas and, after the addition of optional ingredients, water or another suitable carrier/vehicle/diluent will be used to bring the total weight up to 100%. All components in the following examples are Active% of the total composition:

**Table 7**

| **Ingredient** | **Ex. A** | **Ex. B** | **Ex. C** | **Ex. D** | **Ex. E** | **Ex. F** |
|---|---|---|---|---|---|---|
| | | | | | | |
| SE | 3 | 10 | 15 | 5 | 10 | 2 |
| Primary surfactant | 10 | 30 | - | 12 | 15 | 10 |
| co-primary surfactant | 5 | - | - | 3 | 5 | - |
| secondary surfactant | 4 | 8 | 15 | 5 | 5 | 5 |
| Nonionic surfactant | - | - | 30 | 5 | 5 | 5 |
| Solubilizing Solvent | - | 1 | - | - | 5 | 2 |
| antimicrobial agent | - | - | 0.3 | - | - | - |
| preservative | 0.05 | 0.10 | 0.10 | 0.05 | 0.10 | 0.05 |
| Colorant | 0.01 | 0.005 | 0.02 | 0.01 | 0.02 | 0.015 |
| Fragrance | 0.4 | 1.0 | 1.5 | 0.5 | 1.0 | 0.6 |

Formulation levels specified can be understood to vary across a range to produce viscosities from about 200cp to about 6000cp, with the formulator deciding based on how the product is to be packaged and dispensed.

The embodiments and examples described here are illustrative, and do not limit the presently described technology in any way.

## Claims

1. A liquid detergent composition, comprising:
0.1% to 99.9% active weight of the total composition of at least one sulfo-estolide surfactant having the following general Formula 1: 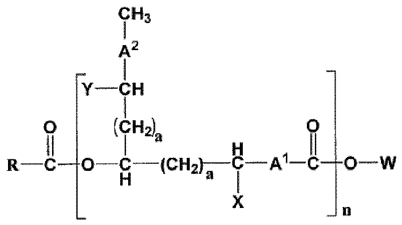
wherein n is an integer from 1-30;
one of X and Y is SO₃-Z, the other of X and Y is H, and X and Y are independently assigned in each repeating unit;
A¹ and A² are linear or branched, saturated or unsaturated, substituted or unsubstituted, alkyl diradicals wherein the total number of carbons for each repeating unit is independent and in the range of C₈ to C₂₂;
a is 0, 1, or 2, and is independently assigned in each repeating unit;
R is linear or branched, saturated or unsaturated, substituted or unsubstituted hydrocarbon wherein the total number of carbon atoms is from 1 to 24;
W is H or a monovalent or divalent metal cation, ammonium cation, substituted ammonium cation, or an alkyl or substituted alkyl group; and
Z is H or a monovalent or divalent metal cation, ammonium or substituted ammonium cation; and
0.1% to 99.9% by weight of at least one additional surfactant.

2. The liquid detergent composition of claim 1, wherein the composition further comprises 0% to 40% active weight of the total composition of at least one foam stabilizing surfactant and/or 1% to 99% of at least one carrier.

3. The liquid detergent composition of any of the preceding claims, wherein the at least one sulfo-estolide surfactant having the general Formula 1 is 0.1% to 50% active weight of the total composition.

4. The liquid detergent composition of any of the preceding claims, wherein the composition is non-toxic, biodegradable, and substantially free of phosphates.

5. The liquid detergent composition of any of the preceding claims, wherein the at least one additional surfactant is 2% to 70% active weight of the total composition.

6. The liquid detergent composition of any of the preceding claims, wherein the at least one additional surfactant is a member selected from the group consisting of at least one anionic surfactant, at least one nonionic surfactant, and combinations thereof.

7. The liquid detergent composition of claim 6, wherein the at least one anionic surfactant is a member selected from the group consisting of sulfoacetates, olefin sulfonates, alkyl benzene sulfonates, alkyl sulfosuccinates, alkyl sulfomethylsuccinates, and combinations thereof.

8. The liquid detergent composition of claim 6, wherein the at least one nonionic surfactant is a member selected from the group consisting of alcohol ethoxylates, alkyl polyglucosides, alkyl ethanolamides, alkyl esters, and combinations thereof.

9. The liquid detergent composition of any of claims 2 to 8, wherein the at least one foam stabilizing surfactant is 0.5% to 15% by active weight of the composition.

10. The liquid detergent composition of any of claims 2 to 9, wherein the at least one foam stabilizing surfactant is a member selected from the group consisting of at least one ampholytic or amphoteric surfactant, at least one nonionic surfactant, and combinations thereof.

11. The liquid detergent composition of claim 10, wherein the at least one ampholytic or amphoteric surfactant is a member selected from the group consisting of amine oxides, amidopropyl amine oxides, betaines, amidopropyl betaines, sulfobetaines, hydroxysultaines, amphoacetates, amphopropionates, alkyl amines, organic diamines, and combinations thereof.

12. The liquid detergent composition of any of the preceding claims, wherein the pH of the composition is from 3 to 10.

13. The liquid detergent composition of any of the preceding claims, wherein the composition further comprises at least one antimicrobial ingredient.

14. The liquid detergent composition of any of the preceding claims, wherein the composition further comprises from 0% to 20% by weight of at least one additive.

15. The liquid detergent composition of any of the preceding claims, wherein the composition further comprises at least one enzyme.

## Patentansprüche

1. Flüssige Detergenszusammensetzung, die umfasst:
0,1% bis 99,9% nach Gewicht der Wirkstoffe der Gesamtzusammensetzung mindestens eines Sulfoestolid-Tensids der allgemeinen Formel 1: 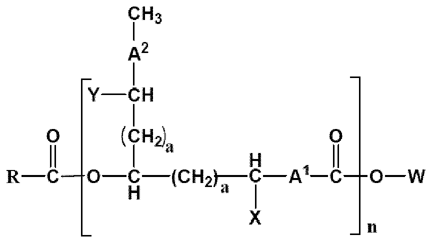
worin n eine ganze Zahl von 1-30 ist;
eines von X und Y SO₃-Z ist, das andere von X und Y H ist, und X und Y in jeder Wiederholungseinheit unabhängig zugeordnet sind;
A¹ und A² lineare oder verzweigte, gesättigte oder ungesättigte, substituierte oder unsubstituierte Alkyldiradikale sind, wobei die Gesamtanzahl von Kohlenstoffatomen für jede Wiederholungseinheit unabhängig ist und in dem Bereich von C₈ bis C₂₂ liegt;
a 0, 1 oder 2 ist und in jeder Wiederholungseinheit unabhängig zugeordnet ist;
R ein linearer oder verzweigter, gesättigter oder ungesättigter, substituierter oder unsubstituierter Kohlenwasserstoff ist, wobei die Gesamtanzahl von Kohlenstoffatomen 1 bis 24 beträgt;
W H oder ein monovalentes oder divalentes Metallkation, Ammoniumkation, substituiertes Ammoniumkation oder eine Alkyl- oder substituierte Alkylgruppe ist; und
Z H oder ein monovalentes oder divalentes Metallkation, Ammonium- oder substituiertes Ammoniumkation ist; und
0,1 % bis 99,9% nach Gewicht mindestens eines zusätzlichen Tensids.

2. Flüssige Detergenszusammensetzung nach Anspruch 1, wobei die Zusammensetzung ferner 0% bis 40% nach Gewicht der Wirkstoffe der Gesamtzusammensetzung mindestens eines schaumstabilisierendes Tensids und/oder 1% bis 99% mindestens eines Trägers umfasst.

3. Flüssige Detergenszusammensetzung nach einem jeglichen der vorhergehenden Ansprüche, wobei das mindestens eine Sulfoestolid-Tensid der allgemeinen Formel 1 in 0,1% bis 50% nach Gewicht der Wirkstoffe der Gesamtzusammensetzung vorliegt.

4. Flüssige Detergenszusammensetzung nach einem jeglichen der vorhergehenden Ansprüche, wobei die Zusammensetzung nicht toxisch, bioabbaubar und im Wesentlichen frei von Phosphaten ist.

5. Flüssige Detergenszusammensetzung nach einem jeglichen der vorhergehenden Ansprüche, wobei das mindestens eine zusätzliche Tensid in 2% bis 70% nach Gewicht der Wirkstoffe der Gesamtzusammensetzung vorliegt.

6. Flüssige Detergenszusammensetzung nach einem jeglichen der vorhergehenden Ansprüche, wobei das mindestens eine zusätzliche Tensid ein Mitglied ist, ausgewählt aus der Gruppe, bestehend aus mindestens einem anionischen Tensid, mindestens einem nicht-ionischen Tensid und Kombinationen davon.

7. Flüssige Detergenszusammensetzung nach Anspruch 6, wobei das mindestens eine anionische Tensid ein Mitglied ist, ausgewählt aus der Gruppe, bestehend aus Sulfoacetaten, Olefinsulfonaten, Alkylbenzolsulfonaten, Alkylsulfosuccinaten, Alkylsulfomethylsuccinaten und Kombinationen davon.

8. Flüssige Detergenszusammensetzung nach Anspruch 6, wobei das mindestens eine nicht-ionische Tensid ein Mitglied ist, ausgewählt aus der Gruppe, bestehend aus Alkoholethoxylaten, Alkylpolyglucosiden, Alkylethanolamiden, Alkylestern und Kombinationen davon.

9. Flüssige Detergenszusammensetzung nach einem jeglichen der Ansprüche 2 bis 8, wobei das mindestens eine schaumstabilisierende Tensid in 0,5% bis 15% nach Gewicht der Wirkstoffe der Zusammensetzung vorliegt.

10. Flüssige Detergenszusammensetzung nach einem jeglichen der Ansprüche 2 bis 9, wobei das mindestens eine schaumstabilisierende Tensid ein Mitglied ist, ausgewählt aus der Gruppe, bestehend aus mindestens einem ampholytischen oder amphoteren Tensid, mindestens einem nicht-ionischen Tensid und Kombinationen davon.

11. Flüssige Detergenszusammensetzung nach Anspruch 10, wobei das mindestens eine ampholytische oder amphotere Tensid ein Mitglied ist, ausgewählt aus der Gruppe, bestehend aus Aminoxiden, Amidopropylaminoxiden, Betainen, Amidopropylbetainen, Sulfobetainen, Hydroxysultainen, Amphoacetaten, Amphopropionaten, Alkylaminen, organischen Diaminen und Kombinationen davon.

12. Flüssige Detergenszusammensetzung nach einem jeglichen der vorhergehenden Ansprüche, wobei der pH-Wert der Zusammensetzung 3 bis 10 beträgt.

13. Flüssige Detergenszusammensetzung nach einem jeglichen der vorhergehenden Ansprüche, wobei die Zusammensetzung ferner mindestens einen antimikrobiellen Bestandteil umfasst.

14. Flüssige Detergenszusammensetzung nach einem jeglichen der vorhergehenden Ansprüche, wobei die Zusammensetzung ferner 0% bis 20% nach Gewicht mindestens eines Additivs umfasst.

15. Flüssige Detergenszusammensetzung nach einem jeglichen der vorhergehenden Ansprüche, wobei die Zusammensetzung ferner mindestens ein Enzym umfasst.

## Revendications

1. Composition détergente liquide, comprenant:
0,1% à 99,9% en poids de la matière active de la composition totale d'au moins un tensioactif sulfo-estolide ayant la Formule générale 1 suivante: 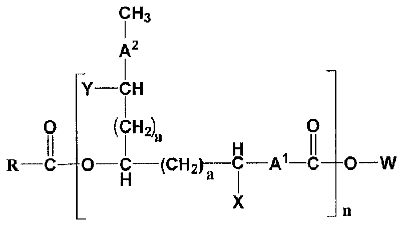
dans laquelle n est un entier de 1-30;
l'un des X et Y est SO₃-Z, l'autre des X et Y est H, et X et Y sont attribués indépendamment dans chaque unité répétitive;
A¹ et A² sont des diradicaux alkyliques linéaires ou ramifiés, saturés ou insaturés, substitués ou non-substitués, dans lesquels le nombre total d'atomes de carbone pour chaque unité répétitive est indépendant et dans l'intervalle de C₈ à C₂₂;
a est 0, 1 ou 2, et est attribué indépendamment dans chaque unité répétitive;
R est un groupe hydrocarboné linéaire ou ramifié, saturé ou insaturé, substitué ou non-substitué, dans lequel le nombre total d'atomes de carbone est de 1 à 24;
W est H ou un cation de métal monovalent ou divalent, un cation ammonium, un cation ammonium substitué, ou un groupe alkyle ou alkyle substitué; et
Z est H ou un cation de métal monovalent ou divalent, un cation ammonium ou un cation ammonium substitué; et
0,1% à 99,9% en poids d'au moins un tensioactif additionnel.

2. Composition détergente liquide selon la revendication 1, dans laquelle la composition comprend en outre 0% à 40% en poids de la matière active de la composition totale d'au moins un tensioactif stabilisateur de mousse et/ou 1% à 99% d'au moins un support.

3. Composition détergente liquide selon l'une quelconque des revendications précédentes, dans laquelle le au moins un tensioactif sulfo-estolide ayant la Formule générale 1 représente 0,1% à 50% en poids de la matière active de la composition totale.

4. Composition détergente liquide selon l'une quelconque des revendications précédentes, dans laquelle la composition est non-toxique, biodégradable, et substantiellement exempte de phosphates.

5. Composition détergente liquide selon l'une quelconque des revendications précédentes, dans laquelle le au moins un tensioactif additionnel représente 2% à 70% en poids de la matière active de la composition totale.

6. Composition détergente liquide selon l'une quelconque des revendications précédentes, dans laquelle le au moins un tensioactif additionnel est un membre choisi dans le groupe consistant en au moins un tensioactif anionique, au moins un tensioactif non-ionique, et leurs combinaisons.

7. Composition détergente liquide selon la revendication 6, dans laquelle le au moins un tensioactif anionique est un membre choisi dans le groupe consistant en sulfoacétates, oléfine sulfonates, alkyl benzène sulfonates, alkyl sulfosuccinates, alkyl sulfométhylsuccinates, et leurs combinaisons.

8. Composition détergente liquide selon la revendication 6, dans laquelle le au moins un tensioactif non-ionique est un membre choisi dans le groupe consistant en éthoxylats d'alcool, alkyl polyglucosides, alkyl éthanolamides, esters alkyliques, et leurs combinaisons.

9. Composition détergente liquide selon l'une quelconque des revendications 2 à 8, dans laquelle le au moins un tensioactif stabilisateur de mousse représente 0,5% à 15% en poids de la matière active de la composition.

10. Composition détergente liquide selon l'une quelconque des revendications 2 à 9, dans laquelle le au moins un tensioactif stabilisateur de mousse est un membre choisi dans le groupe consistant en au moins un tensioactif ampholytique ou amphotère, au moins un tensioactif non-ionique, ou leurs combinaisons.

11. Composition détergente liquide selon la revendication 10, dans laquelle le au moins un tensioactif ampholytique ou amphotère est un membre choisi dans le groupe consistant en oxydes d'amine, oxydes d'amidopropyl amine, bétaïnes, amidopropyl bétaïnes, sulfobétaïnes, hydroxysultaïnes, amphoacétates, amphopropionates, alkyl amines, diamines organiques, et leurs combinaisons.

12. Composition détergente liquide selon l'une quelconque des revendications précédentes, dans laquelle le pH de la composition est de 3 à 10.

13. Composition détergente liquide selon l'une quelconque des revendications précédentes, dans laquelle la composition comprend en outre au moins un composant antimicrobien.

14. Composition détergente liquide selon l'une quelconque des revendications précédentes, dans laquelle la composition comprend en outre de 0% à 20% en poids d'au moins un additif.

15. Composition détergente liquide selon l'une quelconque des revendications précédentes, dans laquelle la composition comprend en outre au moins une enzyme.
